(19) Europäisches Patentamt
European Patent Office
Office européen des brevets

(11) Veröffentlichungsnummer: **0 378 092 B1**

(12) **EUROPÄISCHE PATENTSCHRIFT**

(45) Veröffentlichungstag der Patentschrift: **11.05.94**

(51) Int. Cl.5: **C07D 239/47**, A01N 43/54

(21) Anmeldenummer: **90100079.4**

(22) Anmeldetag: **03.01.90**

Die Akte enthält technische Angaben, die nach dem Eingang der Anmeldung eingereicht wurden und die nicht in dieser Patentschrift enthalten sind.

(54) **Herbizide Sulfonylharnstoffe, Verfahren zu ihrer Herstellung und ihre Verwendung.**

(30) Priorität: **10.01.89 DE 3900472**

(43) Veröffentlichungstag der Anmeldung:
**18.07.90 Patentblatt 90/29**

(45) Bekanntmachung des Hinweises auf die Patenterteilung:
**11.05.94 Patentblatt 94/19**

(84) Benannte Vertragsstaaten:
**BE CH DE DK ES FR GB IT LI NL**

(56) Entgegenhaltungen:
**EP-A- 0 073 627**
**EP-A- 0 141 777**
**EP-A- 0 162 723**
**US-A- 4 547 215**

(73) Patentinhaber: **BASF Aktiengesellschaft**
**Carl-Bosch-Strasse 38**
**D-67063 Ludwigshafen(DE)**

(72) Erfinder: **Hamprecht, Gerhard, Dr.**
**Rote-Turm-Strasse 28**
**D-6940 Weinheim(DE)**
Erfinder: **Westphalen, Karl-Otto, Dr.**
**Mausbergweg 58**
**D-6720 Speyer(DE)**
Erfinder: **Würzer, Bruno, Dr.**
**Rüdigerstrasse 13**
**D-6701 Otterstadt(DE)**

**EP 0 378 092 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft substituierte Sulfonylharnstoffe der allgemeinen Formel I

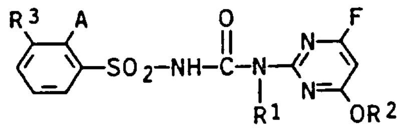

I

in der die Substituenten folgende Bedeutung haben:

- $R^1$ Wasserstoff, eine $C_1$-$C_3$-Alkylgruppe, eine $C_3$-$C_6$-Alkenylgruppe oder eine $C_3$-$C_6$-Alkinylgruppe;
- $R^2$ eine $C_1$-$C_4$-Alkylgruppe;
- $R^3$ Wasserstoff oder ein Halogenatom und
- A ein Halogenatom oder ein Rest

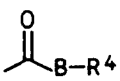

wobei

- B ein Sauerstoffatom oder eine Alkyliminogruppe

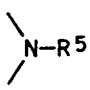

;

- $R^4$ Wasserstoff, eine $C_1$-$C_6$-Alkylgruppe, welche bis zu drei der folgenden Reste tragen kann: Halogen, $C_1$-$C_4$-Alkoxy, $C_1$-$C_4$-Alkylthio, $C_1$-$C_4$-Halogenalkoxy, $C_1$-$C_4$-Alkoxy-$C_1$-$C_2$-alkoxy, $C_3$-$C_7$-Cycloalkyl und/oder Phenyl;
  eine $C_5$-$C_7$-Cycloalkylgruppe, welche bis zu drei $C_1$-$C_4$-Alkylgruppen tragen kann;
  eine $C_3$-$C_6$-Alkenylgruppe oder eine $C_3$-$C_6$-Alkinylgruppe und
- $R^5$ Wasserstoff, eine $C_1$-$C_6$-Alkylgruppe,
  oder gemeinsam mit $R^4$ eine $C_4$-$C_6$-Alkylenkette, worin eine Methylengruppe durch ein Sauerstoffatom oder eine $C_1$-$C_4$-Alkyliminogruppe ersetzt sein kann,
  bedeutet.

Des weiteren betrifft die Erfindung ein Verfahren zur Herstellung der Verbindungen I sowie ihre Verwendung als herbizide Mittel.

Aus der US-A-4 547 215 sind verschiedene Sulfonyl-pyrimidyl-harnstoffe als Herbizide bekannt, welche im Pyrimidylteil durch Chlor substituiert sind. Diese Verbindungen lassen jedoch wegen der geringen Selektivität gegen Schadpflanzen sowie wegen der relativ hohen Aufwandmengen zu wünschen übrig.

Der Erfindung lagen daher neue Verbindungen aus der Klasse der Sulfonyl-pyrimidyl-harnstoffe mit verbesserten herbiziden Eigenschaften als Aufgabe zugrunde.

Entsprechend dieser Aufgabe wurden die eingangs definierten Sulfonylharnstoffe gefunden.

Ferner wurde gefunden, daß die Verbindungen I sowie deren Alkali- und Erdalkalimetallsalze eine gute Selektivität gegen Schadpflanzen in Kulturen wie Mais haben.

Außerdem wurden zum Teil chemisch eigenartige Verfahren zur Herstellung der Verbindungen I gefunden. Insbesondere die Synthese der Vorstufe III bietet gegen den Stand der Technik (J. Med. Chem. 6, 688 (1963); J. Chem. Soc. 6, 1280 (1970)) bei geringerem Aufwand durch eine höhere Regioselektivät und damit bessere Ausbeute, einen entscheidenden Vorteil.

Die erfindungsgemäßen Sulfonylharnstoffe der Formel I sind auf verschiedenen Wegen Zugänglich:

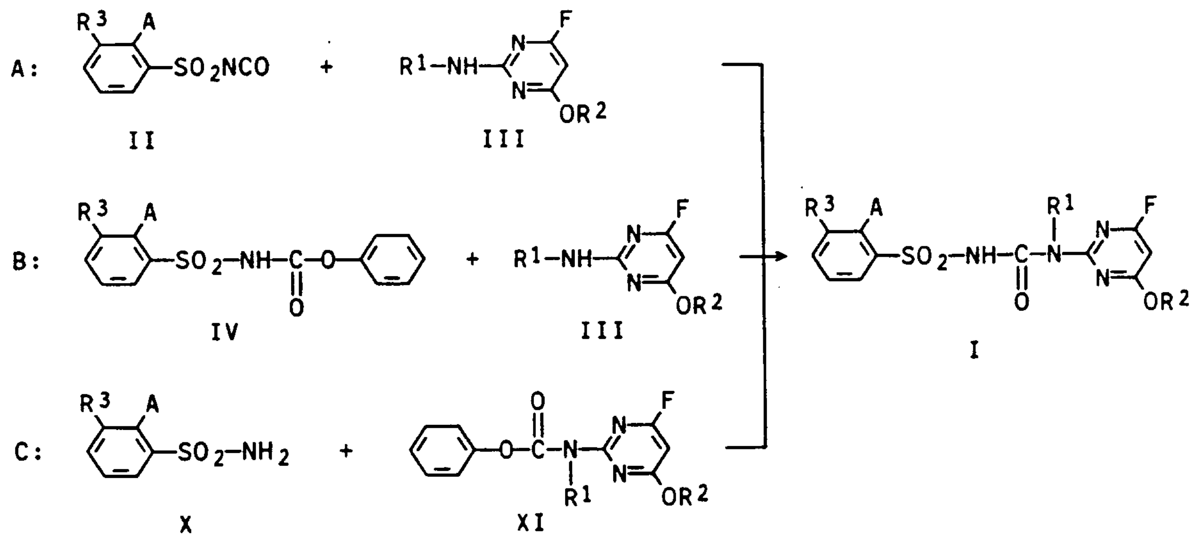


A: Man setzt ein Sulfonylisocyanat II in an sich bekannter Weise (EP-A-162 723) in einem inerten organischen Lösungsmittel mit ungefähr der stöchiometrischen Menge eines 2-Aminopyrimidinderivats III bei einer Temperatur von 0 bis 120 ° C, vorzugsweise 10 bis 100 ° C um. Die Reaktion kann drucklos oder unter Druck (bis 50 bar), vorzugsweise bei 1 bis 5 bar, kontinuierlich oder diskontinuierlich durchgeführt werden.
Geeignete Lösungsmittel sind in der obengenannten Literatur aufgeführt.

B: Man setzt ein entsprechendes Sulfonylcarbamat der Formel IV in an sich bekannter Weise (EP-A-162 723) in einem inerten organischen Lösungsmittel bei einer Temperatur zwischen 0 und 120 ° C, vorzugsweise 10 bis 100 ° C mit einem 2-Aminopyrimidinderivat III um. Es können hierbei Basen wie tertiäre Amine zugesetzt werden, wodurch die Reaktion beschleunigt und die Produktqualität verbessert werden.
Geeignete Basen hierfür sind z.B. tertiäre Amine wie Pyridin, die Picoline, 2,4- und 2,6-Lutidin, 2,4,6-Collidin, p-Dimethylamino-pyridin, 1,4-Diaza[2,2,2]bicyclooctan [DABCO] und 1,8-Diazabicyclo-[5,4,0]-undec-7-en.
zweckmäßig verwendet man als Lösungsmittel die in der Literatur angegebenen und/oder Halogenkohlenwasserstoffe wie Dichlormethan und Chlorbenzol, Ether wie Diethylether, Tetrahydrofuran und Dioxan, Acetonitril, Dimethylformamid und/oder Essigsäureethylester in einer Menge von 100 bis 4000 Gew.%, vorzugsweise von 1000 bis 2000 Gew.%, bezogen auf die Ausgangsstoffe II, IV und X.

Von den erfindungsgemäß benötigten Zwischenprodukten III ist das 2-Amino-4-ethoxy-6-fluorpyrimidin bekannt (J. Med. Chem. 6, 688 (1963)). Es mußte bisher auf aufwendige Weise aus 2-Amino-4,6-difluorpyrimidin und Natriumethylat in trockenem Toluol unter Wechsel des Lösungsmittels und Aufarbeitung in Ether, Einengen und Kristallisation aus einem großen Volumen Petrolether hergestellt werden, wobei aber nur eine Ausbeute von 61 % für das Rohprodukt erreicht wurde.

Noch problematischer ist die Gewinnung des als Ausgangsmaterial benötigten 2-Amino-4,6-difluorpyrimidins, das bei der Aminolyse von 2,4,6-Trifluorpyrimidin in absolutem Ethanol, nach Fällungs- und Waschvorgängen von Rückstand und Filtrat sowie einer Wasserdampfdestillation zur Isomerentrennung in 66 % Ausbeute gewonnen wurde, wobei eine isomere 4-Aminoverbindung sowie Hydrolyseprodukte anfielen.

Ähnlich erhielten R.E. Banks et al. (J. Chem. Soc. 6, 1280, (1970), bei der Aminolyse des Trifluorpyrimidins in wäßrigem Ammoniak bei 0 ° C ein Isomerengemisch mit 53 % Ausbeute an der 2-Amino- und 26 % Ausbeute an der 4-Aminoverbindung.

Im Hinblick auf das ungelöste Isomerenproblem, die erhaltenen Ausbeuten und die Reaktionsbedingungen sind alle bekannten Verfahren bezüglich einfacher und wirtschaftlicher Arbeitsweise, gerade auch im industriellen Maßstab, unbefriedigend.

Im Rahmen der Herstellung der erfindungsgemäßen Verbindungen sind die Zwischenprodukte III jedoch auf wesentlich vorteilhaftere Weise zugänglich:

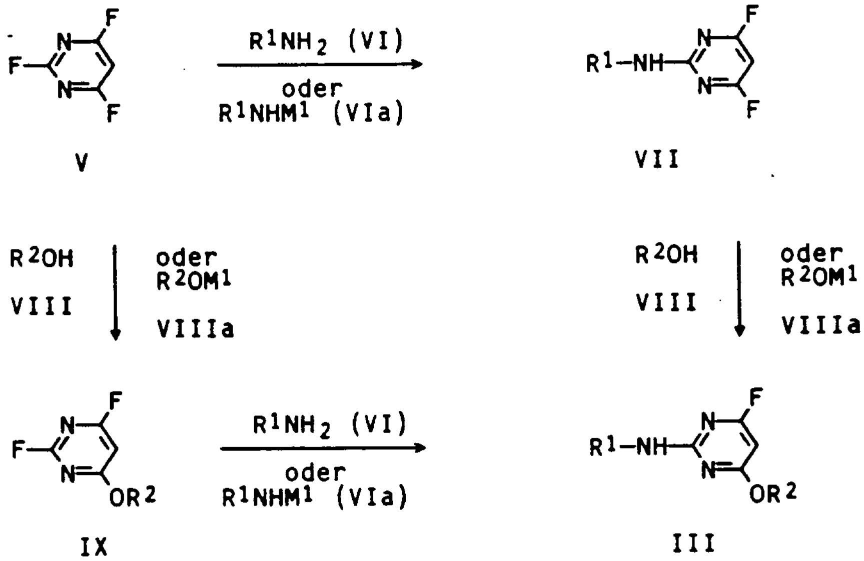


V VII IX III

Die Herstellung der Verbindungen III erfolgt in zwei unabhängigen Reaktionsstufen, wobei die Reihenfolge dieser Reaktionsstufen austauschbar ist.

So kann man beispielsweise 2,4,6-Trifluorpyrimidin in einem aprotischpolaren Lösungsmittel

a) mit einem Amin VI, welches in wäßriger Lösung vorliegen kann, in Anwesenheit oder Abwesenheit einer Base sowie bei zweiphasiger Reaktionsführung in Gegenwart eines Phasentransferkatalysators oder

b) mit einem Aminsalz VIa, in Abwesenheit oder Gegenwart einer Base, sowie bei zweiphasiger Reaktionsführung in Gegenwart eines Phasentransferkatalysators

bei einer Temperatur von -80 bis +20°C umgesetzt werden, wonach das so erhaltene 2-Aminopyrimidinderivat VII ohne Lösungsmittel oder in Anwesenheit eines inerten organischen Lösungsmittels

c) mit einem Alkohol VIII in Anwesenheit oder Abwesenheit einer Base oder

d) mit einem Alkoholat VIIIa in Gegenwart des entsprechenden Alkohols VIII

bei Temperaturen zwischen 0 und 140°C zum Aminopyrimidin III umgesetzt wird. Diese Umsetzungen können drucklos oder unter Druck (1 bis 10 bar, vorzugsweise 1 bis 5 bar), kontinuierlich oder diskontinuierlich durchgeführt werden.

$M^1$ in Formel VIa sowie Formel VIIIa bedeutet jeweils ein Alkalimetallkation wie Lithium-, Natrium- und Kaliumkation oder das Äquivalent eines Erdalkalimetallkations wie Magnesium-, Calcium- und Bariumkation.

Für die Umsetzung des 2,4,6-Trifluorpyrimidins V mit dem Amin $R^1NH_2$ (VI) zu VII sind folgende Lösungsmittel geeignet:

Ether wie Methyl-tert.-butylether, Diethylether, Ethylpropylether, n-Butylethylether, Di-n-butylether, Diisobutylether, Diisoamylether, Di-isopropylether, Cyclohexylmethylether, Tetrahydrofuran, 1,2-Dimethoxyethan, Diethylenglycoldimethylether und Anisol;

chlorierte Kohlenwasserstoffe wie 1,1,2,2-Tetrachlorethan 1,1-Dichlorethylen, Chlorbenzol, 1,2-Dichlorbenzol und 1-Chlornaphthalin sowie entsprechende Gemische.

Zweckmäßig verwendet man das Lösungsmittel in einer Menge von 100 bis 2000 Gew.%, vorzugsweise 500 bis 1500 Gew.%, bezogen auf den Ausgangsstoff V.

Als Säureakzeptor verwendet man vorteilhaft einen Überschuß an Amin oder dessen Alkalimetall- oder Erdalkalimetallsalz. Vorteilhaft gibt man das Amin in einer doppelt äquivalenten Menge oder hiervon einen über- oder Unterschuß von bis zu 15 % bezogen auf den Ausgangsstoff V innerhalb 1 bis 2 Stunden zu einer Mischung von Ausgangsstoff V in einem der vorgenannten Lösungsmittel bei (-80) bis 20°C, vorzugsweise (-30) bis (-10)°C, rührt gegebenenfalls bis zu einer Stunde nach und läßt dann zur Aufarbeitung auf 25°C erwärmen.

Sofern man das primäre Amin $R^1NH_2$ (VI, $R^1 \neq H$) als wäßrige Lösung einsetzt, wird der Reaktionslösung zweckmäßigerweise ein Phasentransferkatalysator zugesetzt.

Hiefür eignen sich beispielsweise Kronenether wie 15-Krone-5 oder 15-Krone-6 bzw. entsprechende benzokondensierte Derivate wie z.B. Dibenzo-18-Krone-6, Polyethylenglykoldialkylether des Typs

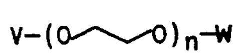

(n = 5 bis 7 und V und W unabhängig voneinander $C_1$-$C_4$-Alkyl) sowie quartäre Ammoniumsalze wie Benzyltriethylammonium-chlorid, Tetrabutylammonium-iodid, Tetrabutylammonium-hydroxid und Tetrabutylammonium-hydrogensulfat.

In diesem Fall wird die Reaktion vorzugsweise bei einer Temperatur zwischen -15 und 20 °C mit einem Katalysatorzusatz von 0,002 bis 0,02 mol-Äquivalenten durchgeführt.

Die Umsetzung der Ausgangsstoffe VII mit VIII führt man zweckmäßig direkt in überschüssigem Alkohol VIII als Lösungsmittel durch. Gegebenenfalls gibt man ein Alkalialkoholat VIIIa in einer äquivalenten Menge oder einem über-oder Unterschuß von bis zu 5 %, bezogen auf den Ausgangsstoff VII, zu einer Suspension des Ausgangsstoffes VII in der 5- bis 20-fachen Gewichtsmenge Alkohol VIII als Lösungsmittel bezogen auf den Ausgangsstoff VII, innerhalb bis zu einer Stunde bei einer Temperatur von 20 bis 80 °C. Zur Beendigung der Umsetzung rührt man dann noch 1/2 bis 8 Stunden bei 0 bis 140 °C, vorzugsweise 20 bis 100 °C nach.

Als basische Verbindungen können beispielsweise Lithium, Natrium, Kalium, Calcium, Barium und Magnesium in Form ihrer Alkoholate benutzt werden; es kommen jedoch auch organische Basen wie Trimethylamin, Triethylamin, N-Ethyldiisopropylamin, Triisopropylamin, N,N-Dimethylamin, N,N-Dimethylcyclohexylamin, N-Methylpyrrolidin, Pyridin, Chinolin, $\alpha$,$\beta$,$\gamma$-Picolin, 2,4-und 2,6-Lutidin und Triethylendiamin in Betracht.

Bei der umgekehrten Reaktionsfolge setzt man 2,4,6-Trifluorpyrimidin (V) in der ersten Stufe entweder mit einem Alkohol VIII oder mit seinem Salz VIIIa zum Pyrimidinderivat IX um, welches anschließend mit einem Amin VI bzw. seinem Salz VIa zum Aminopyrimidinderivat III derivatisiert wird.

Diese Umsetzungen verlaufen analog zu den vorstehend geschilderten Bedingungen in den gleichen Lösungsmitteln bei Verwendung gleicher Säureacceptoren und gleicher Mengenverhältnisse.

Die Reaktionstemperatur liegt bei der Umsetzung von V mit dem Alkohol VIII bzw. seinem Salz VIIIa zwischen -20 und 140 °C, vorzugsweise zwischen 0 und 100 °C, und bei der Umsetzung von IX mit einem Amin VI bzw. seinem Salz VIa zwischen -40 und 100 °C, vorzugsweise zwischen -20 und +20 °C.

Zur Isolierung der Aminopyrimidine III dienen die hierfür in der Literatur üblichen Aufarbeitungsmethoden.

Im Hinblick auf den Stand der Technik liefert das erfindungsgemäße Verfahren in allen Schritten auf einfacherem und wirtschaftlicherem weg die Verbindungen I in vergleichsweise besserer Ausbeute und Reinheit.

C: Man setzt ein Sulfonamid der Formel X in an sich bekannter weise (AP-A-141 777) in einem inerten organischen Lösungsmittel mit ungefähr der stöchiometrischen Menge eines Phenylcarbamats XI bei einer Temperatur von 0 bis 120 °C, vorzugsweise 20 bis 100 °C um. Die Reaktion kann drucklos oder unter Druck (bis 50 bar), vorzugsweise bei 1 bis 5 bar, kontinuierlich oder diskontinuierlich durchgeführt werden.

Geeignete Lösungsmittel sind neben den in der oben zitierten Literatur aufgeführten z.B. Nitrokohlenwasserstoffe wie Nitroethan und Nitrobenzol, Nitrile wie Acetonitril und Benzonitril, Ester wie Essigsäureethylester, Amide wie Dimethylformamid und/oder Ketone wie Aceton.

Bevorzugt wird die Umsetzung in Essigsäureethylester als Lösungsmittel und mit Pyridin oder einem der vorstehend genannten tertiären Amine als Base.

Die als Ausgangsstoffe der Formel IV benötigten Sulfonamide lassen sich aus 6-Halogen-anthranilsäureestern durch Meerwein-Reaktion und anschließende Umsetzung mit Ammoniak herstellen.

Verbindungen der Formel I, in der $R^4$ Wasserstoff bedeutet, erhält man durch Hyrolyse von Estern der Formel I, in der $R^4$ einen $C_1$-$C_6$-Alkylrest bedeutet. Die Hydrolyse wird mit mindestens der doppelten Menge einer Base wie Natrium- oder Kaliumhydroxid, zweckmäßig in einem Lösungsmittelgemisch mit der 2- bis 8-fachen Menge Methanol und der 10- bis 40-fachen Menge Wasser, bezogen auf das Gewicht des entsprechenden Esters der Formel I, bei 30 bis 80 °C während 1 bis 20 Stunden durchgeführt. Durch Ansäuern fällt man die Sulfonamidcarbonsäuren der Formel I aus.

Im Hinblick auf die biologische Wirksamkeit sind Verbindungen der Formel I bevorzugt, in denen die Substituenten folgende Bedeutung haben:

$R^1$ Wasserstoff und Methyl,
$R^2$ Methyl, Ethyl, n-Propyl und Isopropyl,
$R^3$ Wasserstoff, Fluor, Chlor und Brom,
A Chlor, eine Carboxylatgruppe und eine Carboxamidgruppe,
$R^4$ Alkylgruppe wie Methyl, Ethyl, n-Propyl und Isopropyl,
Alkenylgruppe wie Allyl, Methallyl, Crotyl und But-1-en-3-yl,
Alkinylgruppe wie Propargyl, But-1-in-3-yl und But-2-inyl,
Halogenalkylrest wie 2-Chlorethyl, 2-Chlor-n-propyl, 3-Chlor-n-propyl, 1-Chlorbut-2-yl, 2-Chlor-iso-butyl, 4-Chlor-n-butyl, Chlor-tert.-butyl, 3-Chlorprop-2-yl und 2,2,2-Trifluorethyl,
Alkoxyalkylrest wie 2-Methoxyethyl, 2-Ethoxyethyl, 3-Methoxy-n-propyl, 2-Methoxy-n-propyl, 3-Methoxy-n-butyl, 1-Methoxy-but-2-yl, Methoxy-tert.-butyl, Ethoxy-tert.-butyl, 2-Methoxy-n-butyl, 4-Methoxy-n-butyl, 2-Ethoxy-n-propyl, 3-Methoxy-n-prop-2-yl, 2-Ethoxy-n-but-1-yl und 4-Ethoxy-n-butyl,
Alkoxyalkoxyalkylrest wie 2-Methoxy-ethoxy-methyl, 2-(Ethoxy)-ethoxy-methyl, 2-(Propoxy)-ethoxy-methyl, 2-Methoxy-ethoxy-ethyl, 2-(Ethoxy)-ethoxy-ethyl und 2-(Methoxy-methoxy)-ethyl,
Halogenalkoxyalkylrest wie 2-($\beta$-Chlorethoxy)-ethyl, 3-($\beta$-Chlorethoxy)-n-propyl und 3-($\gamma$-Chlor-n-propoxy)-n-propyl,
Cycloalkylrest wie Cyclopentyl und Cyclohexyl,

$R^5$ Wasserstoff,
Alkylgruppe wie Methyl, Ethyl, n-Propyl, Isopropyl und n-Butyl
oder gemeinsam mit $R^4$ Tetramethylen, Pentamethylen, Hexamethylen, Ethylenoxyethylen und Ethylen-N-methylimino-ethylen.

Als Salze der Verbindungen der Formel I kommen landwirtschaftlich brauchbare Salze, beispielsweise Alkalimetallsalze, wie das Kalium- oder Natriumsalz, Erdalkalimetallsalze, wie das Calcium-, Magnesium- oder Bariumsalz, Mangan-, Kupfer-, Zink- oder Eisensalze sowie Ammonium, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Die erfindungsgemäßen herbiziden und wachstumsregulierenden Verbindungen I bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:

I. Man vermischt 90 Gewichtsteile der Verbindung Nr. 1.010 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1.001 werden in einer Mischung gelöst, die aus 80 Gewichtsteilen xylol, 10 Gewichtsteilen des Anlagerungsproduktes von 8 bis 10 Mol Ethylenoxid an 1 Mol ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.002 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 2.006 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des wirkstoffs Nr. 1.009 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 1.058 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 1.059 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 2.004 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

IX. Man vermischt 90 Gewichtsteile der Verbindung Nr. 2.001 mit 10 Gewichtsteilen N-Methyl-α-pyrrolidon und erhält eine Lösung, die zur Anwendung in Form kleinster Tropfen geeignet ist.

X. 20 Gewichtsteile der Verbindung Nr. 1.009 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

XI. 20 Gewichtsteile der Verbindung Nr. 1.001 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

XII. 20 Gewichtsteile des Wirkstoffs Nr. 2.007 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

XIII. 20 Gewichtsteile des Wirkstoffs Nr. 3.001 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer

Hammermühle vermahlen. Durch feines Verteilen der Mischung in 20 000 Gewichtsteilen Wasser erhält man eine Spritzbrühe, die 0,1 Gew.% des Wirkstoffs enthält.

XIV. 3 Gewichtsteile des Wirkstoffs Nr. 1.059 werden mit 97 Gewichtsteilen feinteiligem Kaolin vermischt. Man erhält auf diese Weise ein Stäubemittel, das 3 Gew.% des Wirkstoffs enthält.

XV. 30 Gewichtsteile des Wirkstoffs Nr. 1.009 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

XVI. 20 Gewichtsteile des Wirkstoffs Nr. 1.001 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines phenolsulfonsäure-Formaldehyd-Kondensates und 68 Gewichtsteilen eines paraffinischen Mineralöls innig vermischt. Man erhält eine stabile ölige Dispersion.

Die Applikation der herbiziden und wachstumsregulierenden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff bei Anwendung als Herbizide betragen je nach Bekämpfungsziel, Jahreszeit, Zielpflanzen und Wachstumsstadium 0,001 bis 3, vorzugsweise 0,01 bis 1 kg/ha aktive Substanz (a.S.).

Synthesebeispiele

Die in den nachstehenden Beispielen wiedergegeben Vorschriften wurden unter entsprechender Abwandlung der Ausgangsverbindungen zur Gewinnung weiterer Verbindungen der Formel I benutzt; die erhaltenen Verbindungen sind in den nachfolgenden Tabellen mit physikalischen Angaben aufgeführt; Verbindungen ohne diese Angaben lassen sich aus den entsprechenden Stoffen in analoger Weise aufbauen. Sie lassen aufgrund ihrer nahen strukturellen Beziehungen zu den hergestellen und untersuchten Verbindungen eine gleichartige Wirkung erwarten.

Synthesebeispiele

1. Herstellung der Zwischenprodukte III

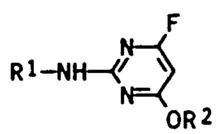

1.1 2-Amino-6-fluor-4-methoxypyrimidin (Variante a)

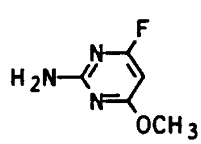

a) 2-Amino-4,6-difluorpyrimidin

69,7 g (4,1 Mol) verflüssigter Ammoniak wurden bei -30 bis -20 °C innerhalb 1 Stunde unter Rühren zu einer Mischung von 250 g (1,865 Mol) 2,4,6-Trifluorpyrimidin in 3,3 l Diethylether gegeben. Zur Aufarbeitung ließ man das Reaktionsgemisch auf 25 °C erwärmen und filtrierte den ausgefallenen Niederschlag ab. Nach dem Waschen mit Ether, Verrühren in Wasser, erneutem Filtrieren und Trocknen wurden 203 g (83 % d. Th.) der Titelverbindung vom Fp. 214-216 °C erhalten. Durch Einengen des Etherfiltrates auf ca. 1/3 seines Volumens konnten weitere 20 g (8 % d. Th.) der Titelverbindung vom Fp. 193-196 °C in einem 1:1-Gemisch mit der isomeren 4-Aminoverbindung isoliert werden.

b) 2-Amino-6-fluor-4-methoxypyrimidin

27 g 30 %iges Natriummethylat (0,15 Mol) wurden innerhalb 20 Minuten unter Rühren bei 65 ° C zu einer Suspension von 19,7 g (0,15 Mol) 2-Amino- 6-difluorpyrimidin in 250 ml abs. Methanol gegeben. Nach 5 Stunden Rühren unter Rückfluß wurde die Reaktionslösung auf 25 ° C abgekühlt, der ausgefallene Niederschlag abgetrennt, mit wenig Methanol gewaschen und dann in Wasser verrührt. Nach dem Abfiltrieren, Waschen mit Wasser und Trocknen erhielt man 16 g (74 % d. Th.) der Titelverbindung vom Fp. 172 ° C. Durch Einengen des Filtrats, Waschen mit Methanol und anschließend mit Wasser wurden weitere 3 g (14 % d. Th.) der Titelverbindung vom Fp. 161-169 ° C isoliert.

1.2 2-Amino-4-ethoxy-6-fluorpyrimidin

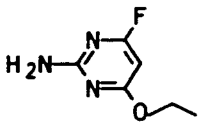

Eine Lösung von 25,2 g (0,3 Mol) Kaliumethylat in 200 ml abs. Ethanol wurde innerhalb 40 Minuten unter Rühren bei 78 ° C zu einer Suspension von 39,3 g (0,3 Mol) 2-Amino-4,6-difluorpyrimidin (1.1a) in 150 ml abs. Ethanol gegeben. Nach 5 Stunden Rühren unter Rückfluß wurde abgekühlt und das Lösungsmittel im Vakuum entfernt. Der Rückstand wurde mit Wasser verrührt, abfiltriert, mit Wasser gewaschen und getrocknet. Man erhielt 39 g (83 % d. Th.) der Titelverbindung vom Fp. 121-123 ° C (vgl. Lit. J. Med. Chem. 6, 688 (1963); 61 % Ausbeute an Rohprodukt; Fp nach Umkristallisation: 120,5-123 ° C).

1.3 2-Amino-6-fluor-4-propyloxypyrimidin

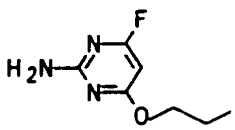

29,4 g (0,3 Mol) Kaliumpropylat wurden analog zu 1.2 mit 39,3 g (0,3 Mol) 2-Amino-4,6-difluorpyrimidin (1.1a) in insgesamt 400 ml n-Propanol umgesetzt. Aus der Reaktionsmischung wurde das Lösungsmittel bei vermindertem Druck entfernt und der Rückstand mit Petrolether gewaschen. Anschließend wurde in Wasser verrührt, abfiltriert, gewaschen und getrocknet, wobei 36,1 g (70 % d. Th.) der Titelverbindung vom Fp. 63-66 ° C anfielen.

1.4 2-Amino-6-fluor-4-isopropyloxypyrimidin

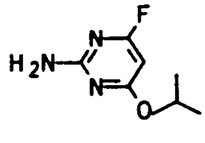

29,4 g (0,3 Mol) Kaliumisopropylat wurden analog zu 1.2 mit 39,3 g (0,3 Mol) 2-Amino-4,6-difluorpyrimidin (1.1a) in insgesamt 400 ml Isopropanol umgesetzt. Nach der Aufarbeitung, Waschen mit Petrolether und Wasser in üblicher Weise erhielt man 38,5 g (75 % d. Th.) der Titelverbindung vom Fp. 66-68 ° C.

1.5 4-Allyloxy-2-amino-6-fluorpyrimidin

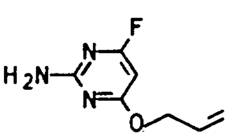

Man gab bei 25 ° C unter Stickstoff-Atmosphäre 1,14 g (0,0382 mol) 80 %iges Natriumhydrid

(Emulsion in Leinöl) zu 70 ml Allylalkohol. Nach 20 Minuten Rühren bei 40 °C wurde diese klare Lösung mit 6,0 g (0,0382 mol) 2-Amino-4,6-difluorpyrimidin (1.1a) versetzt und anschließend 1,5 Stunden bei 97 °C gerührt. Zur Aufarbeitung wurde der überschüssige Alkohol bei vermindertem Druck abdestilliert, der so erhaltene Rückstand in Methylenchlorid aufgenommen, mit Wasser gewaschen, über Magnesiumsulfat getrocknet und anschließend vom Lösungsmittel befreit. Das so erhaltene zähe Öl kristallisierte beim Anreiben mit n-Pentan. Nach dem Abfiltrieren, Waschen mit Wasser und Trocknen erhielt man so 4,6 g (71,2 % d.Th.) der Titelverbindung vom Fp. 62-66 °C.

1.6 2-Amino-6-fluor-4-methoxypyrimidin (Variante b)

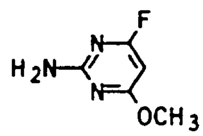

a) 2,4-Difluor-6-methoxypyrimidin

Zu einer Mischung aus 250 g (1,865 mol) 2,4,6-Trifluorpyrimidin in 1,4 l Methanol wurden bei -20 °C innerhalb von 45 Minuten 335,8 g (1,865 mol) 30 %iger Natriummethylat (in Methanol) gegeben und weitere 30 Minuten bei dieser Temperatur gerührt. Anschließend ließ man auf 25 °C erwärmen und engte die Reaktionsmischung auf ca. 1/3 ihres Volumens ein.

Das so erhaltene Gemisch wurde zwischen Diethylether und Wasser verteilt, die organische Phase über Magnesiumsulfat getrocknet und eingeengt. Noch Destillation (1,1 m Kolonne, 3 mm V-Füllkörper) erhielt man 141,6 g (52 % d.Th.) der Titelverbindung von Kp.: 144-145 °C.

Aus dem Destillationssumpf erhält man durch Destillation über einen Normag-Aufsatz 114,4 g (42 % d.Th.) an 4,6-Difluor-2-methoxypyrimidin vom Kp.: 157-161 °C.

b) 2-Amino-6-fluor-4-methoxypyrimidin

13,6 g (0,8 mol) Ammoniak in 30 ml Methyl-tert.-butylether wurden bei -20 bis -10 °C innerhalb 20 Minuten unter Rühren zu einer Mischung von 52 g (0,356 mol) 2,4-Difluor-6-methoxypyrimidin in 300 ml Methyl-tert.-butylether gegeben. Nach weiteren 2 Stunden bei -15 °C und 3 Stunden bei 25 °C wurde der ausgefallene Niederschlag abgesaugt, mit Methyl-tert.-butylether gewaschen, mit Wasser verrührt, abfiltriert, erneut gewaschen und anschließend getrocknet, wobei 36,1 g (71 % d. Th.) der Titelverbindung vom Fp. 171-173 °C anfielen.

1.7 4-Fluor-6-methoxy-2-methylaminopyrimidin

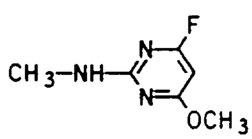

46,8 g einer 40 %igen Methylaminlösung in Wasser (0,605 mol) wurden bei 0 bis 2 °C innerhalb 30 Minuten unter Rühren zu einer Mischung von 41,9 g (0,287 mol) 2,4-Difluor-6-methoxypyrimidin (1.6a) und einer Spatelspitze Triethyl-benzylammoniumchlorid (Phasentransferkatalysator) in 100 ml Methyl-tert.-butylether gegeben. Nach 1 Stunde bei 0 °C und 3 Stunden bei 25 °C wurde die organische Phase abgetrennt, mit Wasser gewaschen und bei vermindertem Druck eingeengt. Der Rückstand wurde mit Pentan verrührt, wobei 39,9 g (88 % d. Th.) 4-Fluor-6-methoxy-2-methylamino-pyrimidin vom Fp. 78-80 °C anfielen.

2. Herstellung der Zwischenprodukte X

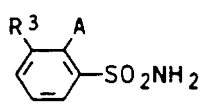

2.1 2-Aminosulfonyl-6-chlor-benzoesäuremethylester

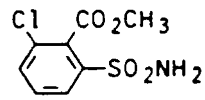

a) 4-Chlor-1,2-benzisothiazol-3-on-1,1-dioxid

504 g (2,02 mol) 6-Chlor-2-aminosulfonyl-benzoesäuremethylester wurden portionsweise zu einer Lösung von 80 g (2,0 mol) Natriumhydroxid in 2,5 l Wasser gegeben, wobei die Temperatur von 25 °C auf 50 °C stieg. Nach 30 Minuten bei dieser Temperatur wurde auf 25 °C abgekühlt, mit Methyl-t-butylether extrahiert und die wäßrige Phase in 2 n Salzsäure eingerührt. Der ausgefallene Niederschlag wurde isoliert, mit Wasser gewaschen und getrocknet. Man erhielt 330 g (75,8 % d.Th.) der Titelverbindung vom Fp.: 210-212 °C.

b) 2-Aminosulfonyl-6-chlor-benzoesäuremethylester

93 g (0,427 mol) 4-Chlor-1,2-benzisothiazol-3-on-1,1-dioxid wurden in 0,8 l Methanol suspendiert und unter Begasen mit Chlorwasserstoff 3 Stunden unter Rückfluß gerührt. Nach dem Abkühlen auf 20 °C, Absaugen und Trocknen wurden 60 g (56,3 % d.Th.) der Titelverbindung vom Fp. 152-153 °C erhalten. Durch Einengen des Filtrates bei vermindertem Druck und Verreiben des Rückstandes mit Methyl-tert.-butylether, erneutem Abfiltrieren und Trocknen erhielt man 42 g (39,4 % d.Th.) einer zweiten Fraktion dieser Verbindung vom Fp. 144-149 °C.

Analog sind z.B. die folgenden Verbindungen erhalten worden:

| | 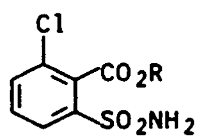 | 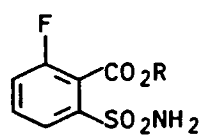 |
|---|---|---|
| R | Fp (°C) | Fp (°C) |
| $C_2H_5$ | 97-101 | 129-131 |
| $n-C_3H_7$ | 111-113 | 104-107 |
| $i-C_3H_7$ | 145-147 | 84- 87 |
| $CH_2CH=CH_2$ | 105-108 | |
| $CH_2CH_2Cl$ | 130-134 | 109-110 |
| $CH_2CH_2OCH_3$ | 102-104 | 135-136 |
| $CH_2CH_2OCH_2CH_2Cl$ | 124-126 | |

2.2 2-Aminosulfonyl-6-fluor-benzoesäuremethylester

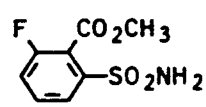

a) 2-Chlorsulfonyl-6-fluor-benzoesäuremethylester

108 g (0,639 mol) 6-Fluoranthranilsäuremethylester und 45 g Natriumnitrit in 106 ml Wasser wurden bei 5 °C unter Rühren getrennt aber gleichzeitig innerhalb 1 Stunde so zu 250 ml konz. Salzsäure gegeben, daß die Esterkomponente im Überschuß vorlag. Nach 20 Minuten Rühren bei 5 bis 8 °C wurde die Reaktionsmischung in einem Guß in eine vorbereitete Lösung von 53 g Schwefeldioxid, 1,7 g Kupfer(II)chlorid in wenig Wasser in 200 ml 1,2-Dichlorethan gegeben und 10 Minuten nachgerührt. Es wurde langsam auf 50 °C erwärmt und unter Einleiten von 46 g Schwefeldioxid 1 1/2 Stunden gerührt. Dann wurde auf 20 °C abgekühlt und während 20 Minuten 5,5 g Chlor unter Rühren eingeleitet. Nach 20 Minuten Rühren wurde die organische Phase

abgetrennt, mit Wasser gewaschen und getrocknet. Man erhielt so 105 g (65 % d.Th.) der Titelverbindung als ein bräunliches Öl.

b) 2-Aminosulfonyl-6-fluor-benzoesäuremethylester

42,5 g Ammoniak wurden bei 20 bis 28 °C unter Rühren in eine Mischung von 252,5 g (1 mol) 2-Chlorsulfonyl-6-fluor-benzoesäuremethylester in 700 ml wasserfreiem Tetrahydrofuran eingegast. Nach einer Stunde Rühren bei 25 °C wurde der ausgefallene Niederschlag abgesaugt, in Wasser gelöst und einmal mit Essigester extrahiert. Beim Ansäuern der wäßrigen Phase mit konz. Salzsäure erhielt man 8,8 g (4,4 % d.Th.) 4-Fluor-1,2-benzisothiazol-3-on-1,1-dioxid vom Fp. 210-212 °C.

Das Tetrahydrofuranfiltrat wurde eingeengt, mit Wasser gewaschen, abfiltriert, mit Diethylether nachgewaschen, erneut abfiltriert und getrocknet. Man erhielt 186 g (79,8 % d.Th.) der Titelverbindung von Fp. 155-159 °C.

3. Herstellung der Zwischenprodukte II

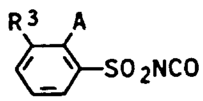

3.1 6-Chlor-2-(isocyanatosulfonyl)benzoesäuremethylester

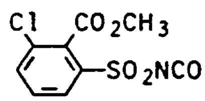

100 g (0,4 mol) 6-Chlor-2-aminosulfonyl-benzoesäuremethylester wurden in 300 ml 1,2-Dichlorethan suspendiert, unter Rühren mit 123 g (1,03 mol) Thionylchlorid versetzt und langsam bis zum Rückfluß erhitzt. Nach 5 Stunden Rühren unter Rückfluß wurde auf 55 °C abgekühlt, 1,5 ml Pyridin zugegeben und unter Einleiten von Phosgen wieder zum Rückfluß erhitzt. Nach 4 Stunden Begasen wurde das Reaktionsgemisch bei vermindertem Druck eingeengt und mit Stickstoff belüftet. Das verbliebene Öl (105 g, 95,2 % d.Th.) wurde ohne weitere Reinigung für die Folgestufen eingesetzt.

4. Herstellung der Wirkstoffe I

4.1 6-Fluor-2-[[(4-fluor-6-methoxy-1,3-pyrimidin-2-yl)aminocarbonyl]-amino -sulfonyl]-benzoesäuremethylester

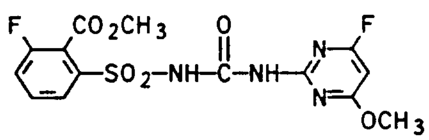

4,3 g (0,03 mol) 6-Fluor-2-isocyanatosulfonyl-benzoesäuremethylester in 15 ml 1,2-Dichlorethan wurden innerhalb 15 Minuten bei 25 °C unter Rühren zu einer Suspension von 4,3 g (0,03 mol) 2-Amino-4-fluor-6-methoxy-pyrimidin (1.1/1.6) in 70 ml 1,2-Dichlorethan gegeben und 12 Stunden bei 25 °C gerührt. Der ausgefallene Niederschlag wurde abgesaugt, zuerst mit 1 n Salzsäure verrührt, mit Wasser gewaschen, dann mit Diethylether verrüht, abgesaugt, gewaschen und getrocknet, wobei 8,1 g (67 % d. Th.) der Titelverbindung vom Fp. 203 °C (Zers.) anfielen (Wirkstoffbeispiel 2.001).

4.2 1-Chlor-2-[[(4-fluor-6-methoxy-1,3-pyrimidin-2-yl]aminocarbonyl]-amino -sulfonyl]-benzol

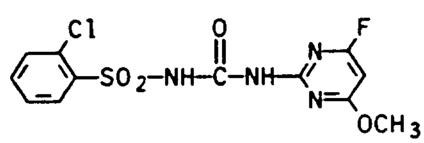

10,9 g (0,05 mol) Chlorbenzol-2-sulfonylisocyanat in 10 ml 1,2-Dichlorethan wurden innerhalb 20 Minuten bei 25 °C unter Rühren zu einer Suspension von 7,15 g (0,05 mol) 2-Amino-4-fluor-6-methoxypyrimidin (1.1/1.6) in 150 ml 1,2-Dichlorethan gegeben und 8 Stunden gerührt. Das Reaktionsgemisch wurde bei vermindertem Druck eingeengt, mit Methyl-tert.-butylether verrührt, abfiltriert und gewaschen. Anschließend wurde mit 2 n Salzsäure verrührt, abfiltriert, mit Wasser gewaschen und getrocknet, wobei 8,6 g (47,7 % d.Th.) Titelverbindung vom Fp. 148-151 °C anfielen (Wirkstoffbeispiel 3.001).

4.3 2-[[(4-Fluor-6-methoxy-1,3-pyrimidin-2-yl)methylaminocarbonyl]-amino-sulfonyl]-benzoesäuremethylester

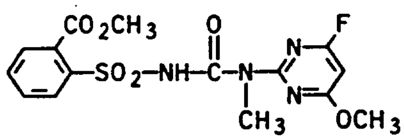

6,3 g (0,04 Mol) der Verbindung 1.7 wurden in 80 ml 1,2-Dichlorethan vorgelegt und unter Rühren bei 25 °C innerhalb 5 Minuten mit 9,6 g (0,04 Mol) 2-Isocyanatosulfonyl-benzoesäuremethylester versetzt. Das Reaktionsgemisch wurde 12 Stunden bei 25 °C gerührt und dann bei vermindertem Druck eingeengt. Der Rückstand wurde in üblicher Weise mit Diethylether/Petrolether 1:1 und mit 1 n Salzsäure sowie mit Wasser behandelt. Nach dem Trocknen erhielt man 10,4 g (65 % d. Th.) der Titelverbindung vom Fp. 148-150 °C (Wirkstoffbeispiel 1.002).

4.4 2-[[(4-Fluor-6-methoxy-1,3-pyrimidin-2-yl)aminocarbonyl]-amino-sulfonyl]-benzoesäureethylester-natriumsalz

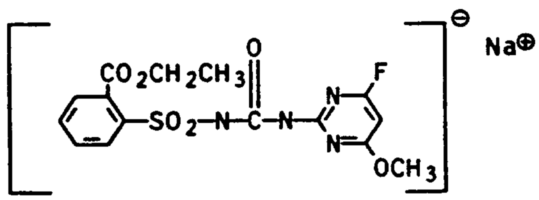

4,2 g (0,0109 Mol) 2-[[(4-Fluor-6-methoxy-1,3-pyrimidin-2-yl)aminocarbonyl]aminosulfonyl]-benzoesäureethylester (Wirkstoffbeispiel 1.009) wurden in 80 ml Methanol suspendiert, bei 25 °C mit 1,9 g (0,0109 Mol) 30 %iger Natriummethylat-Lösung in Methanol versetzt und 10 Minuten gerührt. Nach dem Abdestillieren des Lösungsmittels bei vermindertem Druck erhielt man 4,4 g (99,1 % d.Th.) der Titelverbindung vorn Fp. 175 °C (Zers.) (Wirkstoffbeispiel 1.009 Natrium-Salz).

Tabelle 1

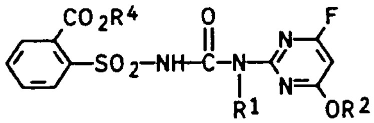

| Nr. | $R^1$ | $R^2$ | $R^4$ | Fp. (°C) |
|---|---|---|---|---|
| 1.001 | H | $CH_3$ | $CH_3$ | 186-188 |
| 1.002 | $CH_3$ | $CH_3$ | $CH_3$ | 148-150 |
| 1.003 | H | $CH_2CH_3$ | $CH_3$ | 169-170 |
| 1.004 | $CH_3$ | $CH_2CH_3$ | $CH_3$ | |
| 1.005 | H | $(CH_2)_2CH_3$ | $CH_3$ | 156-158 |
| 1.006 | $CH_3$ | $(CH_2)_2CH_3$ | $CH_3$ | |
| 1.007 | H | $CH(CH_3)_2$ | $CH_3$ | 134-135 |
| 1.008 | $CH_3$ | $CH(CH_3)_2$ | $CH_3$ | |
| 1.009 | H | $CH_3$ | $CH_2CH_3$ | 165-168<br>175 [Natriumsalz] |
| 1.010 | $CH_3$ | $CH_3$ | $CH_2CH_3$ | 141-146 |
| 1.011 | H | $CH_3$ | $(CH_2)_2CH_3$ | 143-147 |
| 1.012 | $CH_3$ | $CH_3$ | $(CH_2)_2CH_3$ | |
| 1.013 | H | $CH_3$ | $CH(CH_3)_2$ | 172-175 |
| 1.014 | $CH_3$ | $CH_3$ | $CH(CH_3)_2$ | |
| 1.015 | H | $CH_3$ | $CH_2CH=CH_2$ | |
| 1.016 | $CH_3$ | $CH_3$ | $CH_2CH=CH_2$ | |
| 1.017 | H | $CH_3$ | $CH_2C\equiv CH$ | |
| 1.018 | $CH_3$ | $CH_3$ | $CH_2C\equiv CH$ | |
| 1.019 | H | $CH_3$ | $CH_2CH=CHCH_3$ | |
| 1.020 | $CH_3$ | $CH_3$ | $CH_2CH=CHCH_3$ | |
| 1.021 | H | $CH_3$ | $CH_2C\equiv CCH_3$ | |
| 1.022 | $CH_3$ | $CH_3$ | $CH_2C\equiv CCH_3$ | |
| 1.023 | H | $CH_3$ | $CH_2C(CH_3)=CH_2$ | |
| 1.024 | $CH_3$ | $CH_3$ | $CH_2C(CH_3)=CH_2$ | |
| 1.025 | H | $CH_3$ | $(CH_2)_2Cl$ | 168-170 |
| 1.026 | $CH_3$ | $CH_3$ | $(CH_2)_2Cl$ | |
| 1.027 | H | $CH_3$ | $(CH_2)_2OCH_3$ | 154-156 |
| 1.028 | $CH_3$ | $CH_3$ | $(CH_2)_2OCH_3$ | |
| 1.029 | H | $CH_3$ | $(CH_2)_2O(CH_2)_2OCH_3$ | |
| 1.030 | $CH_3$ | $CH_3$ | $(CH_2)_2O(CH_2)_2OCH_3$ | |
| 1.031 | H | $CH_3$ | $CH_2O(CH_2)_2OCH_2CH_3$ | |
| 1.032 | $CH_3$ | $CH_3$ | $CH_2O(CH_2)_2OCH_2CH_3$ | |

Tabelle 1 (Fortsetzung)

| Nr. | $R^1$ | $R^2$ | $R^4$ | Fp. (°C) |
|---|---|---|---|---|
| 1.033 | H | $CH_3$ | $CH_2CF_3$ | |
| 1.034 | $CH_3$ | $CH_3$ | $CH_2CF_3$ | |
| 1.035 | H | $CH_3$ | $C(CH_3)_2CH_2OCH_3$ | |
| 1.036 | $CH_3$ | $CH_3$ | $C(CH_3)_2CH_2OCH_3$ | |
| 1.037 | H | $CH_3$ | $CH_2O(CH_2)_2OCH_3$ | |
| 1.038 | $CH_3$ | $CH_3$ | $CH_2O(CH_2)_2OCH_3$ | |
| 1.039 | H | $CH_3$ | Cyclopentyl | |
| 1.040 | $CH_3$ | $CH_3$ | Cyclopentyl | |
| 1.041 | H | $CH_3$ | Cyclohexyl | |
| 1.042 | $CH_3$ | $CH_3$ | Cyclohexyl | |
| 1.043 | H | $CH_3$ | $(CH_2)_2SCH_3$ | |
| 1.044 | $CH_3$ | $CH_3$ | $(CH_2)_2SCH_3$ | |
| 1.045 | H | $CH_3$ | $(CH_2)_2OCH_2CF_3$ | |
| 1.046 | $CH_3$ | $CH_3$ | $(CH_2)_2OCH_2CF_3$ | |
| 1.047 | H | $CH_3$ | $CH_2C_6H_5$ | |
| 1.048 | $CH_3$ | $CH_3$ | $CH_2C_6H_5$ | |
| 1.049 | H | $CH_3$ | $(CH_2)_3CH_3$ | |
| 1.050 | $CH_3$ | $CH_3$ | $(CH_2)_3CH_3$ | |
| 1.051 | H | $CH_3$ | $CH_2CH(CH_3)_2$ | |
| 1.052 | $CH_3$ | $CH_3$ | $CH_2CH(CH_3)_2$ | |
| 1.053 | H | $CH_3$ | $C(CH_3)_3$ | |
| 1.054 | $CH_3$ | $CH_3$ | $C(CH_3)_3$ | |
| 1.055 | H | $CH_3$ | $CH(CH_3)CH_2CH_3$ | |
| 1.056 | $CH_3$ | $CH_3$ | $CH(CH_3)CH_2CH_3$ | |
| 1.057 | H | $CH_3$ | $(CH_2)_2OCH_3$ | 142 (Zers.) [Natriumsalz] |
| 1.058 | H | $CH_3$ | $(CH_2)_2Cl$ | 152 (Zers.) [Natriumsalz] |
| 1.059 | H | $CH_3$ | $CH_3$ | 144 (Zers.) [Natriumsalz] |
| 1.060 | H | $CH_3$ | $CH_3$ | 164 (Zers.) [Calciumsalz] |
| 1.061 | H | $CH_3$ | $CH(CH_3)_2$ | 184 (Zers.) [Natriumsalz] |
| 1.062 | H | $CH_3$ | $(CH_2)_2CH_3$ | 186 (Zers.) [Natriumsalz] |

Tabelle 2

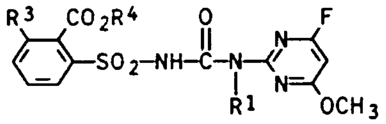

| Nr. | $R^1$ | $R^3$ | $R^4$ | Fp. (°C) |
|---|---|---|---|---|
| 2.001 | H | F | $CH_3$ | 203 (Zers.) |
| 2.002 | $CH_3$ | F | $CH_3$ | 152-155 |
| 2.003 | H | F | $CH_2CH_3$ | |
| 2.004 | $CH_3$ | F | $CH_2CH_3$ | 138-140 |
| 2.005 | H | F | $(CH_2)_2CH_3$ | |
| 2.006 | $CH_3$ | F | $(CH_2)_2CH_3$ | 142-144 |
| 2.007 | H | Cl | $CH_3$ | 222-225 |
| 2.008 | $CH_3$ | Cl | $CH_3$ | |
| 2.009 | H | Cl | $CH_2CH_3$ | 185-188 |
| 2.010 | $CH_3$ | Cl | $CH_2CH_3$ | |
| 2.011 | H | Cl | $(CH_2)_2CH_3$ | |
| 2.012 | $CH_3$ | Cl | $(CH_2)_2CH_3$ | |
| 2.013 | H | Br | $CH_3$ | 220-225 (Zers.)<br>185 (Zers.)<br>Natriumsalz |
| 2.014 | $CH_3$ | Br | $CH_3$ | |
| 2.015 | H | Br | $CH_2CH_3$ | |
| 2.016 | $CH_3$ | Br | $CH_2CH_3$ | |
| 2.017 | H | Br | $(CH_2)_2CH_3$ | |
| 2.018 | $CH_3$ | Br | $(CH_2)_2CH_3$ | |
| 2.019 | H | Cl | $CH(CH_3)_2$ | |
| 2.020 | $CH_3$ | Cl | $CH(CH_3)_2$ | |
| 2.021 | H | F | $CH(CH_3)_2$ | 194-196 |
| 2.022 | $CH_3$ | F | $CH(CH_3)_2$ | |
| 2.023 | H | F | $CH_2CH=CH_2$ | |
| 2.024 | $CH_3$ | F | $CH_2CH=CH_2$ | |
| 2.025 | H | Cl | $CH_2CH=CH_2$ | 116-120 |
| 2.026 | $CH_3$ | Cl | $CH_2CH=CH_2$ | |
| 2.027 | H | Cl | $(CH_2)_2O(CH_2)_2Cl$ | 148-151<br>180 (Zers.)<br>Natriumsalz |
| 2.028 | $CH_3$ | Cl | $(CH_2)_2O(CH_2)_2Cl$ | |
| 2.029 | H | F | $(CH_2)_2O(CH_2)_2Cl$ | |
| 2.030 | $CH_3$ | F | $(CH_2)_2O(CH_2)_2Cl$ | |

Tabelle 3

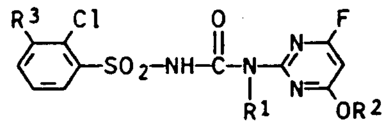

| Nr. | $R^1$ | $R^2$ | $R^3$ | Fp. (°C) |
|---|---|---|---|---|
| 3.001 | H | $CH_3$ | H | 148-151 |
| 3.002 | $CH_3$ | $CH_3$ | H | 116-118 |
| 3.003 | H | $CH_3$ | Cl | |
| 3.004 | $CH_3$ | $CH_3$ | Cl | |
| 3.005 | H | $CH_2CH_3$ | H | |
| 3.006 | $CH_3$ | $CH_2CH_3$ | H | |
| 3.007 | H | $CH_2CH_3$ | Cl | |
| 3.008 | $CH_3$ | $CH_2CH_3$ | Cl | |
| 3.009 | H | $(CH_2)_2CH_3$ | H | |
| 3.010 | $CH_3$ | $(CH_2)_2CH_3$ | H | |
| 3.011 | H | $(CH_2)_2CH_3$ | Cl | |
| 3.012 | $CH_3$ | $(CH_2)_2CH_3$ | Cl | |
| 3.013 | H | $CH(CH_3)_2$ | H | |
| 3.014 | $CH_3$ | $CH(CH_3)_2$ | H | |
| 3.015 | H | $CH(CH_3)_2$ | Cl | |
| 3.016 | $CH_3$ | $CH(CH_3)_2$ | Cl | |
| 3.017 | H | $(CH_2)_3CH_3$ | H | |
| 3.018 | $CH_3$ | $(CH_2)_3CH_3$ | H | |
| 3.019 | H | $(CH_2)_3CH_3$ | Cl | |
| 3.020 | $CH_3$ | $(CH_2)_3CH_3$ | Cl | |

Tabelle 4

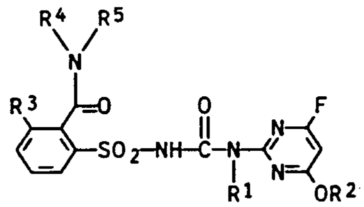

| Nr. | $R^1$ | $R^2$ | $R^3$ | $R^4$ | $R^5$ | Fp. (°C) |
|---|---|---|---|---|---|---|
| 4.001 | H | $CH_3$ | H | $CH_3$ | H | |
| 4.002 | $CH_3$ | $CH_3$ | H | $CH_3$ | H | |
| 4.003 | H | $CH_2CH_3$ | H | $CH_3$ | H | |
| 4.004 | $CH_3$ | $CH_2CH_3$ | H | $CH_3$ | H | |
| 4.005 | H | $(CH_2)_2CH_3$ | H | $CH_3$ | H | |
| 4.006 | $CH_3$ | $(CH_2)_2CH_3$ | H | $CH_3$ | H | |
| 4.007 | H | $CH_3$ | F | $CH_3$ | H | |
| 4.008 | $CH_3$ | $CH_3$ | F | $CH_3$ | H | |
| 4.009 | H | $CH_3$ | F | $CH_3$ | $CH_3$ | 178-179<br>192(Zers.)<br>[Natriumsalz] |
| 4.010 | $CH_3$ | $CH_3$ | F | $CH_3$ | $CH_3$ | |
| 4.011 | H | $CH_3$ | Cl | $CH_3$ | H | |
| 4.012 | $CH_3$ | $CH_3$ | Cl | $CH_3$ | H | |
| 4.013 | H | $CH_3$ | Cl | $CH_3$ | $CH_3$ | |
| 4.014 | $CH_3$ | $CH_3$ | Cl | $CH_3$ | $CH_3$ | |
| 4.015 | H | $CH_3$ | Br | $CH_3$ | H | |
| 4.016 | $CH_3$ | $CH_3$ | Br | $CH_3$ | H | |
| 4.017 | H | $CH_3$ | Br | $CH_3$ | $CH_3$ | |
| 4.018 | $CH_3$ | $CH_3$ | Br | $CH_3$ | $CH_3$ | |
| 4.019 | H | $CH_3$ | H | $CH_3$ | $CH_3$ | |
| 4.020 | $CH_3$ | $CH_3$ | H | $CH_3$ | $CH_3$ | |
| 4.021 | H | $CH_3$ | H | $CH_2CH_3$ | H | |
| 4.022 | $CH_3$ | $CH_3$ | H | $CH_2CH_3$ | H | |
| 4.023 | H | $CH_3$ | H | $(CH_2)_2CH_3$ | H | |
| 4.024 | $CH_3$ | $CH_3$ | H | $(CH_2)_2CH_3$ | H | |
| 4.025 | H | $CH_3$ | H | $CH(CH_3)_2$ | H | |
| 4.026 | $CH_3$ | $CH_3$ | H | $CH(CH_3)_2$ | H | |
| 4.027 | H | $CH_3$ | F | $CH_2CH_3$ | H | |
| 4.028 | $CH_3$ | $CH_3$ | F | $CH_2CH_3$ | H | |
| 4.029 | H | $CH_3$ | F | $(CH_2)_2CH_3$ | H | |
| 4.030 | $CH_3$ | $CH_3$ | F | $(CH_2)_2CH_3$ | H | |
| 4.031 | H | $CH_3$ | F | $CH(CH_3)_2$ | H | |
| 4.032 | $CH_3$ | $CH_3$ | F | $CH(CH_3)_2$ | H | |

Tabelle 4 (Fortsetzung)

| Nr. | $R^1$ | $R^2$ | $R^3$ | $R^4$ | $R^5$ | Fp. (°C) |
|---|---|---|---|---|---|---|
| 4.033 | H | $CH_3$ | Cl | $CH_2CH_3$ | H | |
| 4.034 | $CH_3$ | $CH_3$ | Cl | $CH_2CH_3$ | H | |
| 4.035 | H | $CH_3$ | Cl | $(CH_2)_2CH_3$ | H | |
| 4.036 | $CH_3$ | $CH_3$ | Cl | $(CH_2)_2CH_3$ | H | |
| 4.037 | H | $CH_3$ | Cl | $CH(CH_3)_2$ | H | |
| 4.038 | $CH_3$ | $CH_3$ | Cl | $CH(CH_3)_2$ | H | |
| 4.039 | H | $CH_3$ | H | $CH_2CH_3$ | $CH_3$ | |
| 4.040 | $CH_3$ | $CH_3$ | H | $CH_2CH_3$ | $CH_3$ | |
| 4.041 | H | $CH_3$ | H | $CH_2CH_3$ | $CH_2CH_3$ | |
| 4.042 | H | $CH_3$ | F | $CH_2CH_3$ | $CH_2CH_3$ | |
| 4.043 | H | $CH_3$ | H | $(CH_2)_2CH_3$ | $(CH_2)_2CH_3$ | |
| 4.044 | H | $CH_3$ | F | $(CH_2)_2CH_3$ | $(CH_2)_2CH_3$ | |
| 4.045 | H | $CH_3$ | H | $(CH_2)_3CH_3$ | $(CH_2)_3CH_3$ | |
| 4.046 | H | $CH_3$ | F | $(CH_2)_3CH_3$ | $(CH_2)_3CH_3$ | |
| 4.047 | H | $CH_3$ | H | $(CH_2)_2OCH_3$ | H | |
| 4.048 | H | $CH_3$ | F | $(CH_2)_2OCH_3$ | H | |
| 4.049 | H | $CH_3$ | H | $(CH_2)_2OCH_3$ | $CH_3$ | |
| 4.050 | H | $CH_3$ | F | $(CH_2)_2OCH_3$ | $CH_3$ | |
| 4.051 | H | $CH_3$ | H | $CH_2CH=CH_2$ | H | |
| 4.052 | H | $CH_3$ | F | $CH_2CH=CH_2$ | H | |
| 4.053 | H | $CH_3$ | H | $CH_2CH=CH_2$ | $CH_3$ | |
| 4.054 | H | $CH_3$ | F | $CH_2CH=CH_2$ | $CH_3$ | |
| 4.055 | H | $CH_3$ | H | $(CH_2)_2O(CH_2)_2OCH_3$ | H | |
| 4.056 | H | $CH_3$ | F | $(CH_2)_2O(CH_2)_2OCH_3$ | H | |
| 4.057 | H | $CH_3$ | H | $-(CH_2)_4-$ | | |
| 4.058 | H | $CH_3$ | F | $-(CH_2)_4-$ | | |
| 4.059 | $CH_3$ | $CH_3$ | H | $-(CH_2)_4-$ | | |
| 4.060 | $CH_3$ | $CH_3$ | F | $-(CH_2)_4-$ | | |
| 4.061 | H | $CH_3$ | H | $-(CH_2)_5-$ | | |
| 4.062 | $CH_3$ | $CH_3$ | H | $-(CH_2)_5-$ | | |
| 4.063 | H | $CH_3$ | F | $-(CH_2)_5-$ | | |
| 4.064 | $CH_3$ | $CH_3$ | F | $-(CH_2)_5-$ | | |
| 4.065 | H | $CH_3$ | H | $-(CH_2)_6-$ | | |
| 4.066 | $CH_3$ | $CH_3$ | H | $-(CH_2)_6-$ | | |
| 4.067 | H | $CH_3$ | F | $-(CH_2)_6-$ | | |
| 4.068 | $CH_3$ | $CH_3$ | F | $-(CH_2)_6-$ | | |
| 4.069 | H | $CH_3$ | H | $-(CH_2)_2O(CH_2)_2-$ | | |
| 4.070 | $CH_3$ | $CH_3$ | H | $-(CH_2)_2O(CH_2)_2-$ | | |
| 4.071 | H | $CH_3$ | F | $-(CH_2)_2O(CH_2)_2-$ | | |

Tabelle 4 (Fortsetzung)

| Nr. | R1 | R2 | R3 | R4 | R5 | Fp. (°C) |
|---|---|---|---|---|---|---|
| 4.072 | $CH_3$ | $CH_3$ | F | | $-(CH_2)_2O(CH_2)_2-$ | |
| 4.073 | H | $CH_3$ | H | | $-(CH_2)_2NCH_3(CH_2)_2-$ | |
| 4.074 | $CH_3$ | $CH_3$ | H | | $-(CH_2)_2NCH_3(CH_2)_2-$ | |
| 4.075 | H | $CH_3$ | F | | $-(CH_2)_2NCH_3(CH_2)_2-$ | |
| 4.076 | $CH_3$ | $CH_3$ | F | | $-(CH_2)_2NCH_3(CH_2)_2-$ | |

Anwendungsbeispiele:

Die herbizide Wirkung der Sulfonylharnstoffe der Formel I auf das Wachstum der Testpflanzen wird durch folgende Gewächshausversuche gezeigt.

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt flach eingesät.

Zum Zwecke der Nachauflaufbehandlung werden entweder direkt gesäte oder in den gleichen Gefäßen aufgewachsene Pflanzen ausgewählt oder sie werden erst als Keimpflanzen getrennt angezogen und einige Tage vor der Behandlung in die Versuchsgefäße verpflanzt.

Je nach Wuchsform werden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt werden, behandelt. Die Aufwandmenge für die Nachauflaufbehandlung beträgt 0,125 kg/ha a.S.

Die Versuchsgefäße werden im Gewächshaus aufgestellt, wobei für wärmeliebende Arten wärmere Bereiche (20 bis 35 °C ) und für solche gemäßigter Klimate 10 bis 20 °C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Lateinischer Name | Deutscher Name |
|---|---|
| Amaranthus retroflexus | Zurückgekrümmter Fuchsschwanz |
| Brassica napus | Winterraps |
| Chrysanthenum corinarium | Kronenwunderblume |
| Cyperus esculentus | Erdmandel |
| Echinochloa crus-galli | Hühnerhirse |
| Malva neglecta | Wegmalve |
| Matricaria inodora | Duftlose Kamille |
| Sinapis alba | Weißer Senf |
| Solanum nigrum | Schwarzer Nachtschatten |
| Stellaria media | Vogelmiere |
| Veronica spp. | Ehrenpreisarten |
| Triticum aestivum | Sommerweizen |

Mit 0,125 kg/ha a.S. im Nachauflaufverfahren oder mit 0,125 kg/ha a.S. im Vorauflaufverfahren eingesetzt, lassen sich mit dem Beispiel 1.001 unerwünschte Pflanzen sehr gut bekämpfen.

Außerdem lassen sich mit 0,15 kg/ha a.S. der Verbindungen 1.009, 1.058 und 1.059 breitblättrige unerwünschte Pflanzen sehr gut bekämpfen, ohne daß Weizen als Beispielkultur nennenswert geschädigt wird.

## Patentansprüche

1. Substituierte Sulfonylharnstoffe der allgemeinen Formel I

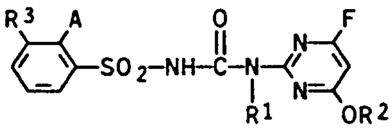

I

in der die Substituenten folgende Bedeutung haben:

$R^1$ Wasserstoff, eine $C_1$-$C_3$-Alkylgruppe, eine $C_3$-$C_6$-Alkenylgruppe oder eine $C_3$-$C_6$-Alkinylgruppe;

$R^2$ eine $C_1$-$C_4$-Alkylgruppe;

$R^3$ Wasserstoff oder ein Halogenatom und

A ein Halogenatom oder einen Rest

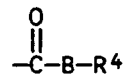

wobei

B ein Sauerstoffatom oder eine Alkyliminogruppe

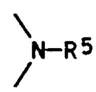

$R^4$ Wasserstoff, eine $C_1$-$C_6$-Alkylgruppe, welche bis zu drei der folgenden Reste tragen kann: Halogen, $C_1$-$C_4$-Alkoxy, $C_1$-$C_4$-Alkylthio, $C_1$-$C_4$-Halogenalkoxy, $C_1$-$C_4$-Alkoxy-$C_1$-$C_2$-alkoxy, $C_3$-$C_7$-Cycloalkyl und/oder Phenyl;
eine $C_5$-$C_7$-Cycloalkylgruppe, welche bis zu drei $C_1$-$C_4$-Alkylgruppen tragen kann;
eine $C_3$-$C_6$-Alkenylgruppe oder eine $C_3$-$C_6$-Alkinylgruppe und

$R^5$ Wasserstoff, eine $C_1$-$C_6$-Alkylgruppe,
oder gemeinsam mit $R^4$ eine $C_4$-$C_6$-Alkylenkette, in der eine Methylengruppe durch ein Sauerstoffatom oder eine $C_1$-$C_4$-Alkyliminogruppe ersetzt sein kann,

bedeutet, sowie deren umweltverträgliche Salze.

2. Sulfonylharnstoffe nach Anspruch 1, in denen die Substituenten folgende Bedeutung haben:

$R^1$ Wasserstoff oder eine Methylgruppe,

$R^2$ eine $C_1$-$C_4$-Alkylgruppe,

$R^3$ Wasserstoff oder ein Halogenatom und

A einen Rest $-CO_2R^4$ worin

$R^4$ eine $C_1$-$C_4$-Alkylgruppe bedeutet.

3. Verfahren zur Herstellung der Verbindungen I gemäß Anspruch 1, in denen A nicht die Bedeutung -COOH besitzt, dadurch gekennzeichnet, daß man ein Sulfonylisocyanat II

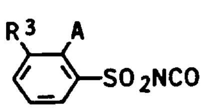

II

in an sich bekannter Weise in einem inerten organischen Lösungsmittel mit der ungefähr stöchiometrischen Menge eines 2-Aminopyrimidinderivats III

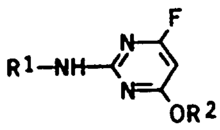

III

umsetzt.

4. Verfahren zur Herstellung der Verbindungen I gemäß Anspruch 1, dadurch gekennzeichnet, daß man ein Carbamat der Formel IV

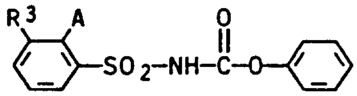

IV

in an sich bekannter Weise in einem inerten organischen Lösungsmittel bei einer Temperatur zwischen 0 und 120 °C mit ungefähr der stöchiometrischen Menge eines 2-Aminopyrimidins III umsetzt.

5. Verfahren nach Anspruch 3 und 4, dadurch gekennzeichnet, daß man die Verbindung III in der Weise herstellt, daß man 2,4,6-Trifluorpyrimidin (V)

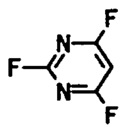

V

in einem aprotisch-polaren organischen Lösungsmittel in Gegenwart einer Base mit einem Amin $R^1NH_2$ (VI) oder mit einem Aminsalz $R^1NHM^1$ (VIa), worin $M^1$ ein Alkalimetallkation oder ein Äquivalent eines Erdalkalimetallkations bedeutet, bei (-80) bis +20 °C zum 2-Amino-4,6-difluorpyrimidin VII

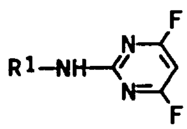

VII

umsetzt, und diese Verbindung bei 0 bis 140 °C in Anwesenheit oder Abwesenheit einer Base mit einem Alkohol $R^2OH$ (VIII) oder mit einem Alkoholat $R^2OM^1$ (VIIIa) zu III umsetzt, oder daß man V zunächst mit VIII oder VIIIa in den Ether IX

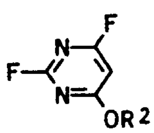

IX

überführt und diesen anschließend mit VI oder VIa zu III umsetzt.

6. 2-[[(4-Fluor-6-methoxy-1,3-pyrimidin-2-yl)aminocarbonyl]-aminosulfonyl]-benzoesäuremethylester

7. Herbizides Mittel, enthaltend einen Sulfonylharnstoff der Formel I gemäß Anspruch 1 oder sein Salz sowie hierfür übliche Trägerstoffe.

8. Verfahren zur Bekämpfung unerwünschten Pflanzenwuchses, dadurch gekennzeichnet, daß man einen Sulfonylharnstoff der Formel I gemäß Anspruch 1 oder eines seiner Salze in einer herbizid wirksamen Menge auf die Pflanzen und/oder ihren Lebensraum einwirken läßt.

**9.** Verfahren zur Bekämpfung unerwünschten Pflanzenwachstums in Mais, dadurch gekennzeichnet, daß man eine herbizid wirksame Menge von 2-[[(4-Fluor-6-methoxy-1,3-pyrimidin-2-yl)aminocarbonyl]-aminosulfonyl]-benzoesäuremethylester verwendet.

**10.** Verwendung der Verbindungen I gemäß Anspruch 1 als herbizide Mittel.

**11.** Verwendung von 2-[[4-Fluor-6-methoxy-1,3-pyrimidin-2-yl)aminocarbonyl]aminosulfonyl]-benzoesäuremethylester nach Anspruch 9 als herbizides Mittel in Maiskulturen.

**12.** Verfahren zur Regulierung des Pflanzenwuchses, dadurch gekennzeichnet, daß man eine wuchsregulierende Menge eines Sulfonylharnstoffs der Formel I gemäß Anspruch 1 oder einer seiner Salze auf die Samen, die Pflanzen und/oder deren Lebensraum einwirken läßt.

**Claims**

**1.** A substituted sulfonylurea of the general formula I

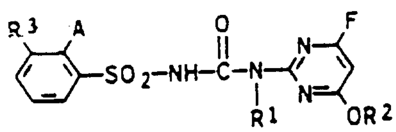

I

in which the substituents have the following meaning:

$R^1$ is hydrogen, $C_1$-$C_3$-alkyl, $C_3$-$C_6$-alkenyl or $C_3$-$C_6$ alkynyl; $R_2$ is $C_1$-$C_4$-alkyl; $R^3$ is hydrogen or halogen and A is halogen or a radical

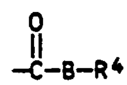

where B is oxygen or an alkylimino group

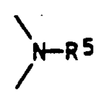

$R^4$ is hydrogen, $C_1$-$C_6$-alkyl which can carry up to three of the following radicals: halogen, $C_1$-$C_4$-alkoxy, $C_1$-$C_4$-alkylthio, $C_1$-$C_4$-haloalkoxy, $C_1$-$C_4$-alkoxy-$C_1$-$C_2$-alkoxy, $C_3$-$C_7$-cycloalkyl and/or phenyl; $C_5$-$C_7$-cycloalkyl which can carry up to three $C_1$-$C_4$-alkyls; $C_3$-$C_6$-alkenyl or $C_3$-$C_6$-alkynyl, and $R^5$ is hydrogen, $C_1$-$C_6$-alkyl, or together with $R^4$ is a $C_4$-$C_6$-alkylene chain in which one methylene can be replaced by oxygen or $C_1$-$C_4$-alkylimino,

or an environmentally compatible salt thereof.

**2.** A sulfonylurea as claimed in claim 1, in which the substituents have the following meaning: $R^1$ is hydrogen or methyl, $R^2$ is $C_1$-$C_4$-alkyl, $R^3$ is hydrogen or halogen and A is $-CO_2R^4$ where $R^4$ is $C_1$-$C_4$-alkyl.

**3.** A process for preparing a compound I as claimed in claim 1, where A is not -COOH, which comprises reacting a sulfonyl isocyanate II

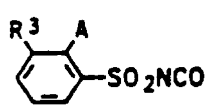

II

in a conventional manner with approximately the stoichiometric amount of a 2-aminopyrimidine derivative III

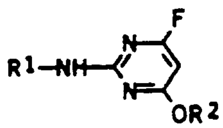

III

in an inert organic solvent.

**4.** A process for preparing a compound I as claimed in claim 1, wherein a carbamate of the formula IV

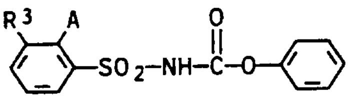

IV

is reacted in a conventional manner with approximately the stoichiometric amount of a 2-aminopyrimidine III in an inert organic solvent at from 0 to 120 °C.

**5.** A process as claimed in claim 3 or 4, wherein a compound III is prepared by reacting 2,4,6-trifluoropyrimidine (V)

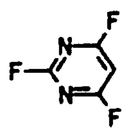

V

in an aprotic polar organic solvent in the presence of a base with an amine $R^1NH_2$ (VI) or with an amine salt $R^1NHM^1$ (VIa), in which $M^1$ is an alkali metal cation or one equivalent of an alkaline earth metal cation, at from -80 to +20 °C to give the 2-amino-4,6-difluoropyrimidine VII

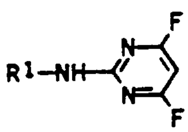

VII

and reacting this compound at 0 to 140 °C in the presence or absence of a base with an alcohol $R^2OH$ (VIII) or with an alcoholate $R^2OM^1$ (VIIIa) to give III, or by converting V initially with VIII or VIIIa into the ether IX

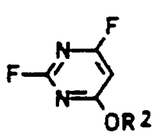

IX

and then reacting the latter with VI or VIa to give III.

**6.** Methyl 2-[N-(4-fluoro-6-methoxy-2-pyrimidinyl)-aminocarbonylaminosulfonyl]benzoate.

**7.** A herbicide containing a sulfonylurea of the formula I as claimed in claim 1, or a salt thereof, and conventional carriers therefor.

**8.** A method for controlling unwanted plant growth, wherein a herbicidally effective amount of a sulfonylurea of the formula I as claimed in claim 1, or of a salt thereof, is allowed to act on the plants and/or their habitat.

**9.** A method for controlling unwanted plant growth in Indian corn, wherein a herbicidally effective amount of methyl 2-(N-(4-fluoro-6-methoxy-2-pyrimidinyl)aminocarbonylaminosulfonyl]benzoate is used.

**10.** The use of a compound I as claimed in claim 1 as a herbicide.

**11.** The use of methyl 2-[N-(4-fluoro-6-methoxy-2-pyrimidinyl)aminocarbonylaminosulfonyl]benzoate as claimed in claim 9 as a herbicide in Indian corn.

**12.** A method for regulating plant growth, wherein a growth-regulating amount of a sulfonylurea of the formula I as claimed in claim 1, or of a salt thereof, is allowed to act on the seeds, the plants and/or their habitat.

**Revendications**

**1.** Sulfonylurées substituées de la formule générale I

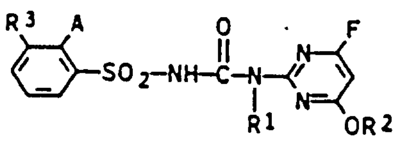

I

dans laquelle les substituants ont la signification suivante
R1, hydrogène, un groupe alkyle en C1-C3, un groupe alcényle en C3-C6 ou un groupe alcynyle en C3-C6
R2, un groupe alkyle en C1-C4
R3, hydrogène ou un atome d'halogène et
A, un atome d'halogène ou un reste

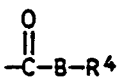

B représentant un atome d'oxygène ou un groupe alkylimine

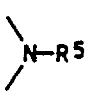

R4, hydrogène, un groupe alkyle en C1-C6, qui peut porter jusqu'à trois des restes suivants : halogène, alcoxy en C1-C4, alkylthio en C1-C4, halogénalcoxy en C1-C4, alcoxy en C1-C4-alcoxy en C1-C2, cycloalkyle en C3-C7 et/ou phényle
un groupe cycloalkyle en C5-C7, qui peut porter jusqu'à trois groupe alkyle en C1-C4;
un groupe alcényle en C3-C6 ou un groupe alkinyle en C3-C6, et
R5, hydrogène, un groupe alkyle en C1-C6
ou, ensemble avec R4, une chaîne alkylène en C4-C6, dans laquelle un groupe méthylène peut être remplacé par un atome d'oxygène ou un groupe alkyle en C1-C4-imino
ainsi que leurs sels acceptables pour l'environnement.

**2.** Sulfonylurées selon la revendication 1 dans lequelles les substituants ont les significations suivantes :
R1, hydrogène ou un groupe méthyle

R2, un groupe alkyle en C1-C4,
R3, hydrogène ou un atome d'halogène et
A, un reste-$CO_2$R4, où R4 représente un groupe alkyle en C1-C4.

3. Procédé de préparation des composés I selon la revendication 1 dans lesquels A ne représente pas -COOH, caractérisé par le fait que l'on fait réagir un sulfonylisocyanate II

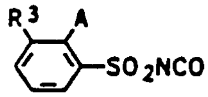

II

de manière connue en soi, dans un solavant organique inerte, avec la quantité à peu près stoechiométrique d'un dérivé de 2-amino-pyrimidine III

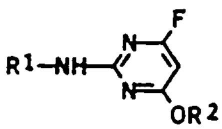

III

4. Procédé de préparation des composés I selon la revendication 1, caractérisé par le fait que l'on fait réagir un carbonate de formule IV

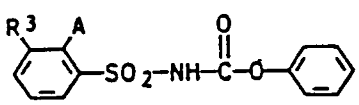

IV

de manière connue en soi, dans un solvant organique inerte, à une température comprise entre 0 et 120 °C
avec la quantité à peu près stoechiométrique d'un dérivé de 2-amino-pyrimidine III.

5. Procédé selon les revendications 3 et 4, caractérisé par le fait qu'on prépare le composé III de la manière suivante :
on fait réagir 2,4,6-trifluoropyrimidine (V)

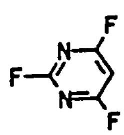

V

dans un solvant organique aprotique-polaire, en présence d'une base avec une amine $R^1NH_2$ (VI) ou un sel d'amine $R^1NHM^1$ (VIa), où $M^1$ représente un cation de métal alcalin ou un équivalent d'un cation de métal alcalino-terreux, entre (-80) et + 20 °C, pour obtenir la 2-amino-4,6-difluoro-pyrimidine VII

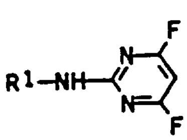

VII

et, pour obtenir III, on fait réagir ce composé entre 0 et 140 °C, en présence ou en l'absence d'une base avec un alcool $R^2OH$ (VIII) ou avec un alcoolate $R^2OM^1$ (VIIIa), ou bien on transforme V d'abord

avec VIII ou VIIIa en l'éther IX

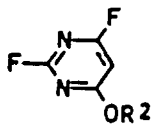

IX

et on obtient III par réaction ensuite de celui-ci avec VI ou VIa.

**6.** Ester méthylique d'acide 2-[[4-fluoro-6-méthoxy-1,3-pyrimidine-2-yl)aminocarbonyl]-aminosulfonyl]-benzoique.

**7.** Herbicide contenant une sulfonylurée de formule I selon la revendication 1 ou son sel ainsi que des véhicules usuels pour cela.

**8.** Procédé de lutte contre la croissance des plantes indésirables, caractérisé par le fait que l'on fait agir une sulfonylurée de formule I selon la revendication 1 ou un de ses sels, en quantité à activité herbicide sur les plantes et/ou leur biotope.

**9.** Procédé de lutte contre la croissance de plantes indésirables dans le maïs, caractérisé par le fait que l'on utilise une quantité efficace au point de vue herbicide d'ester méthylique d'acide 2-[[4-fluoro-6-méthoxy-1,3-pyrimidine-2-yl)aminocarbonyl]-aminosulfonyl-benzoique.

**10.** Utilisation des composés I selon la revendication 1 comme herbicides.

**11.** Utilisation d'ester méthylique d'acide 2-[[4-fluoro-6-méthoxy-1,3-pyrimidine-2-yl)aminocarbonyl]-aminosulfonyl-benzoique selon la revendication 9 comme herbicide dans la culture du maïs.

**12.** Procédé de régularisation de la croissance des plantes, caractérisé par le fait que l'on fait agir une quantité régularisant la croissance d'une sulfonylurée de formule I selon la revendication 1 ou un de ses sels, sur les semences, les plantes et/ou leur biotope.